# EUROPEAN PATENT APPLICATION

(11) **EP 2 264 460 A1**
(43) Date of publication of application: **22.12.2010**
(21) Application number: 09163160.6
(22) Date of filing: 18.06.2009
(51) Int. Cl.: G01N 33/543, G01N 33/553

(54) **Device having self-assembled-monolayer**

(71) Applicant: NXP B.V., 5656 AG Eindhoven (NL); Hardingham, Christopher Mark, Redhill, Surrey RH1 1DL (GB)
(72) Inventor: Merelle, Thomas, Redhill, Surrey RH1 1DL (GB); Lambert, Magali, Redhill, Surrey RH1 1DL (GB); Frederix, Filip, Redhill, Surrey RH1 1DL (GB)
(74) Representative: Hardingham, Christopher Mark

(57) **Abstract**

A device for bio-sensing applications is disclosed, comprising a substrate such as a semiconductor chip having Cu electrodes thereon, and a self assembled monolayer bonded to at least one of the Cu electrodes, wherein molecules of the self-assembled monolayer comprise a head group which bonds to Cu, a carbon-comprising chain comprising a chain of at least 12 C atoms, and a terminal group which is hydrophilic and for binding a bio-receptor. The terminal group is hydrophilic to allow binding to the bio-receptor, and inclusion of the carbon-comprising chain, limits or avoids corrosion of the copper.

Also disclosed is a method of providing such a device, activating the terminal group and coupling a bio-receptor to the activated terminal group. Disclosure further extends to use of such a device for bio-sensing applications.

## Description

### Field of the Invention

This invention relates to devices comprising substrates such as semiconductor chips having copper electrodes with self-assembled monolayers bonded thereto, and is particularly related to such devices which are intended for bio-sensing applications. It further relates to methods of manufacturing such devices, and to uses of such devices.

### Background of the Invention

Recently, the present Applicant has disclosed a bio-sensing device, based on capacitive (or resistive or inductive - in general impedance) sensing of a bio-molecule, which bio-molecule is mechanically coupled to a micro-electrode. (Patent Application Publication WO-A-2009-047703). The device is particularly suited to miniaturisation and can be integrated both with conventional semiconductor technology and with micro-fluidic technology for efficient, high sensitivity and rapid throughput bio-sensing.

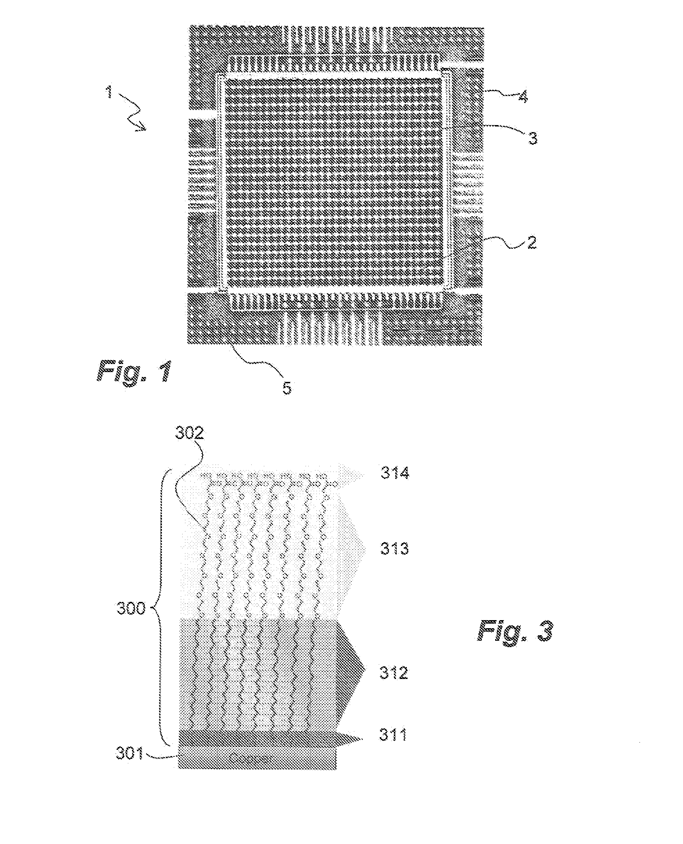

In order to bind the target bio-molecule (e.g. viruses, DNA, RNA, proteins, antigen, peptides, etc...) or biomarker to the microelectrode with adequate selectivity a bio-receptor molecule is utilised such as an antibody, peptide, single chain Fv, antibody fragment, aptamers, DNA, biological receptors, or the like. The bio-receptor is linked to the microelectrode by means of a self-assembled-monolayer (SAM), as shown in Figure 1.

In contrast to conventional semiconductor devices, the bio-sensing device requires that the semiconductor device typically be exposed to a liquid environment with a relatively high salt concentration. (As an example, a physiological salt concentration is around 150mM). Conventional semiconductor devices normally are designed and packaged to exclude moisture, liquid and oxygen, since it is well known that moisture, liquid or oxygen can oxidise the materials of the device, in particular metallic contact materials, and thereby degrade the device performance and even result in catastrophic device failure.

Also, bio-sensing devices may be the subject of potentially conflicting design choices. One example of such a conflicting design choice is that for the micro-electrodes between copper, which is suitable for CMOS semiconductor processing, and gold, which is convenient for SAM adhesion. Another potentially conflicting design choice for the SAM is between a hydrophilic molecule, which is good for subsequent bonding to a bio-receptor, and hydrophobic, which is good for realising a barrier layer towards oxidation and moisture sensitive micro/nano electrodes.

SAMs are well known. Mainly, they have been developed for application on gold surfaces rather than copper. Gold is a noble metal, which does not rapidly oxidize and is readily compatible for biomolecules (such as antibodies and DNA). Moreover, gold is more bio-compatible than copper. The interface chemistry on gold surfaces is therefore more straightforward than on metals which are easily oxidized such as copper. Unfortunately, though, gold is not semiconductor compatible - at least not as far as the overwhelmingly most commonly used semiconductor, silicon, is concerned. Its diffusion into silicon creates deep levels in the silicon band gap which damages the carrier (electron and hole) properties of silicon-base semiconductor materials.

Partly as a result, there is interest in developing SAMs for use on copper electrodes, despite the fact the copper is less convenient than gold. Thanks to its high electrical conductivity, good scalability and heat conduction, copper is nowadays widely used for back-end metallization in silicon-based semiconductor process technologies such as CMOS technology. However, the disadvantage of copper is that copper oxidizes very easily in saline solutions, which are often used in biosensing experiments and in real clinical samples. In particular, SAMs having thiol (-SH) functional groups do not bond well to oxidised copper, since the underlying oxide is not stable. An unstable oxide can give rise to under-etching phenomena which could drastically affect the SAM stability on copper; furthermore, oxidation during the lifetime of a bio-sensor can result in electrical and/or mechanical degradation of the device.

In order to protect the copper electrode from oxidation, the SAM should contain a hydrophobic part.

It is known, for example from Hutt & Lui (Applied Surface Science 252 (2005) 400-411) to provide SAMs on Copper that can give a degree of protection against oxidation. Such a SAM has also been disclosed by Jennings et al (Journal Am. Chem. Soc. 125 (2003) 2950-2957), in which the SAM has a thiol (HS) group, bonding to the copper electrode, and a long-chain alkoxy-alkane group, which provides a barrier to oxidation.

Known SAMs for bonding to copper are mostly hydrophobic: a hydrophobic SAM is easier to deposit on copper surface since its molecules have only one functional group (i.e. the bonding group such as thiol [-SH]) and do not suffer from potential "flipping" problems of hydrophilic SAMs made of molecules containing two functional groups at their extremities i.e. a bonding group such as thiol (-SH) and a functional group such as carboxylic acid (-COOH) to bind a bio-receptor. Flipping occurs when the "wrong" end of the SAM molecule - that is, the supposed to functional group - bonds to the copper electrode. It is known that COOH groups can bind to inorganic oxides such as oxidised copper electrodes: this can lead to flipping of the interface molecules so the for instance thiol group is at the top and result in inactive antibody binding. In addition, these flipping phenomena could lead to unstructured SAMs, resulting in stability problems, both since the packing would then be poor and there would be potential for "both" ends of the molecule to bind to the copper to form a bridge.

In contrast, it is desirable for bio-sensing applications, that the SAM is hydrophilic, since the SAM provides the interface chemistry to bind the bio-receptors. The functional groups should allow attachment of bio-receptors but also avoid non-specific adsorption. Although various functional groups satisfy this requirement, they are characterised in the common theme that they are hydrophilic by nature. Hydrophilic functional groups do not suffer from the problems related to hydrophobic surfaces: that is, non-specific binding and leading to denaturation of the bio-receptors.

Known thiol-alkane (or thiol alkoxy-alkane) SAMs bonded onto copper are thus unsuitable for use in biosensing applications. There is thus an ongoing need for devices which allow SAMs to be used to couple bio-receptor molecules to copper electrode for bio-sensing applications.

### Summary of the invention

It is an object of the present invention to provide a device which is compatible both with copper electrodes for conventional semiconductor processing and is suitable for binding to the bioreceptors. It is a further object of the present invention to provide a method of producing such a device. It is yet further object of the invention to provide a means of bio-sensing.

According to an aspect of the present invention there is provided a device for bio-sensing applications, comprising a substrate having Cu electrodes thereon, and a self assembled monolayer bonded to at least one of the Cu electrodes, wherein molecules of the self-assembled monolayer comprise a head group which bonds to Cu, a carbon-comprising chain comprising a chain of at least 11 C atoms, and a terminal group which is hydrophilic and for binding a bio-receptor. The substrate may for instance be a semiconductor chip.

In embodiments molecules of the self-assembled monolayer further comprise at least one polyethylene oxide group between the alkyl chain and the terminal group. Beneficially, the presence of such polyethylene oxide group in the SAM acts to reduce non-specific adsorption during bio-sensing measurements. The polyethylene oxide groups decrease the hydrophobic and/or electrostatic interactions between the surface (that is, the SAM plus bio-receptors) and the analytes/target molecules of interest within the analyzed sample. It is therefore useful to prevent false positive results.

In embodiments, the molecules of the self-assembled monolayer are selected from the group consisting in HS-R1-Y and HS-R1-(-O-CH₂-CH₂-)ₘ-Y, HSe-R1-Y and HSe-R1-(-O-CH₂-CH₂-)ₘ-Y, where m is either a positive integer or 0. Both the thiol chemical moiety HS and the selenol chemical moiety HSe are capable of strong and effective bonds to copper electrodes. Preferably, Y is selected from the group consisting in -(COOH), -OH, -(CHO), -biotin, -cyclic ether, and amine -(NH₂),

In embodiments, R1 is a carbon-comprising chain of n carbon atoms, interrupted by p hetero-atoms, where n and p are each positive integers. In other embodiments, R1 is a carbon-comprising chain of n carbon atoms without interruption, where n is a positive integer.

In embodiments, R1 comprises an alkyl, alkenyl, cyclic alkyl, aryl, alkyl bound to aryl, alkenyl bound to aryl or alkynyl bound to aryl. In a particularly preferred embodiment, R1 is an alkyl group. In the case that the alkyl chain has no side chains it is particularly suited for close packing and thereby it provides an effective barrier between the terminal group of the SAM and the copper electrode to limit or even prevent corrosion inhibition and thus to protect the copper.

In embodiments n is an integer greater than 10; in preferred embodiments n is an integer between 13 and 19. Such values for n have been found experimentally to provide effective corrosion inhibition and copper protection properties between the terminal group and the copper electrode, whilst being reasonably practicable to synthesize without undue difficulty or cost, capable of processing without undue tangling, and in the more convenient liquid state.

According to another aspect of the present invention, there is provided a sensor comprising a device as described above, wherein the sensor further comprises a bio-receptor bonded to the terminal group.

Preferably, but without limitation, the bio-receptor is bound to the terminal group by means of a cross-linker, which cross-linker is selected from the group consisting in 1-Ethyl-3-[3-dimethylaminopropyl]-carbodiimide hydrochloride, N-hydroxide succinimide, biotin hydrazide, 2-(2-pyridinyldithio)-ethane amine (PDEA), Maleimide-R-NH₂ and their derivatives containing e.g. polyethylene oxide units. These cross-linkers are known to be particularly useful or specific bioreceptor immobilisation; however, as will be readily apparent to the skilled person, alternative cross-linkers are included within the scope of the invention.

According to a further aspect of the present invention, there is provided a method of forming a biosensor comprising providing a device as described above, activating the terminal group, and coupling a bio-receptor to the activated terminal group.

In embodiments, the coupling and activation are effected in a buffer solution.

According to a yet further aspects of the present invention, the invention extends to use of a sensor as described above to detect a predetermined biologically active molecule or a predetermined biologically active functional group.

In embodiments, the respective active target molecule or molecular group is in a buffer solution. Although not required for the invention, it is generally considered necessary to synthesise, process, and to transport bio-receptor materials in a buffer solution, which typically is saline. Since it is saline, it conducts current. Alternatively, without limitation the target moledcule of molecular group may be in biological fluid such as urine, blood, serum or the like, which also contain salts, and thus are (or can be made to be) electrically conductive. It is necessary that the solution conducts current, since in the detection of binding of target bio-molecules, an electrode is immersed into the carrier fluid and an AC bias applied to the electrode. The sensor's transistor phase and amplitude changes during the measurement are signatures of the chemical binding between the target bio-molecule and its specific bio-receptor because of the sudden change in capacitance and resistance in the cavity.

These and other aspects of the invention will be apparent from, and elucidated with reference to, the embodiments described hereinafter.

### Brief description of Drawings

Embodiments of the invention will be described, by way of example only, with reference to the drawings, in which
Fig. 1 shows a schematic of an array of devices, for use in bio-sensing applications;
Fig. 2 shows a schematic cross-section through a device of figure 1, including an activated self-assembled monolayer, with a bio-receptor molecule bound thereto;
Fig. 3 shows schematically, an example of a monolayer on a copper substrate, in accordance with the invention;
Fig. 4 shows the chemical processes in activating and coupling a bio-receptor molecule to a SAM on a copper substrate;
Fig. 5 show the chemical chains of exemplary SAM and intermediate molecules; and
Fig. 6 show, in schematic cross-section, the effect of a loosely-deposited SAM (at (a) and (b)) and a tightly-packed SAM, (at (c) and (d)), respectively before and after exposure to a electrochemical stress;

It should be noted that the Figures are diagrammatic and not drawn to scale. Relative dimensions and proportions of parts of these Figures have been shown exaggerated or reduced in size, for the sake of clarity and convenience in the drawings. The same reference signs are generally used to refer to corresponding or similar feature in modified and different embodiments

### Detailed description of embodiments

An idealised schematic plan view of a semiconductor chip for use in bio-sensing applications is shown in figure 1. The chip 1 comprises an array 3, in this case a Cartesian x-y array, of individual sensor electrodes 2, which is surrounded by peripheral region 4 where copper dummies are buried in the silicon oxide 5. In use (not shown in this figure) microfluidic channels are provided which allow for a fluid to flow over the surface of the chip. In bio-sensing applications, the fluid may carry, in solution or suspension, target bio-molecules. The copper nano/microelectrode can bind the receptor biomolecules capable of binding the target biomolecules in the fluid.

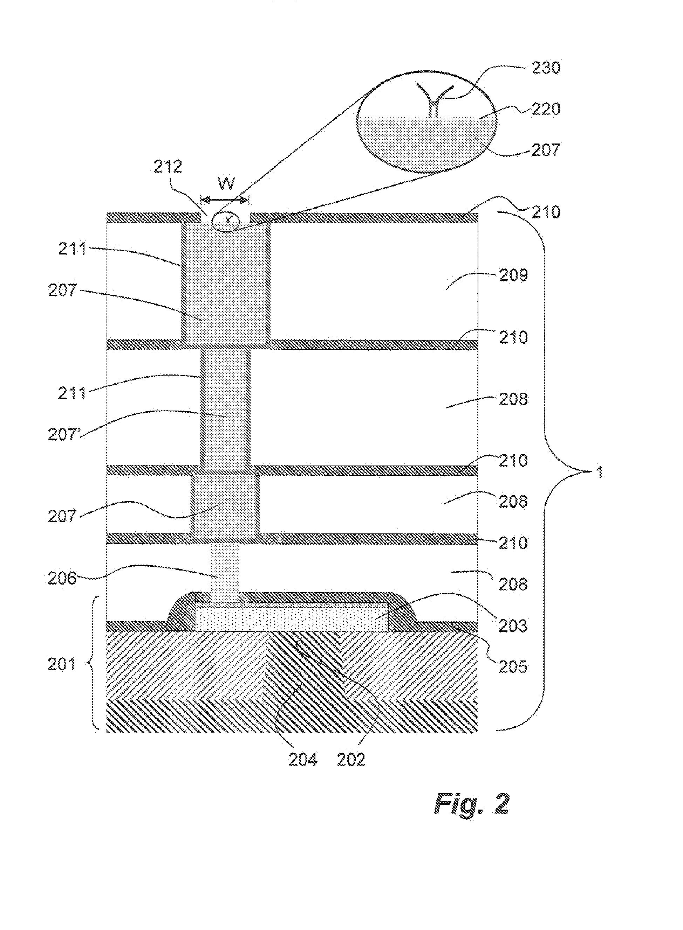

Figure 2 shows a schematic cross-section of a part of a semiconductor chip as shown in Figure 1. The chip 1 includes a conventional transistor MOSFET 201 comprising a channel 202, in an n-well or a p-well 204 and underneath a polysilicon gate 203. The gate oxide between the channel and the gate is not shown in the figure. A silicon carbide spacer layer 205 lies over the gate 205 as well as the source and drain (not shown). The electrical connection to the gate is provided by means of tungsten contact 206. Above the transistor are multiple layers of protective silicon oxide 208, spaced apart by further silicon carbide barrier layers 210. The silicon carbide barrier layers 210 define multiple metallisation layers for the device, with vias therebetween. The gate contact 206 is in electrical connection to the surface of the chip by means of copper 207, 207' in tracks in the metallisation layers, and as well as in the vias therebetween. The tracks of copper 207 and the vias of copper 207' are lined by tantalum nitride liners 211.

At the surface of the chip, overlying the topmost silicon oxide layer 209, is a final silicon carbide barrier. The silicon carbide barrier also partially overlays the topmost copper metallisation at 207, and has therein an aperture 212 exposing the copper metal. The width W of the aperture may typically be of the order of 250 nm.

As shown in the insert to figure 2, overlying the copper in the aperture 212 is a thin layer 220, comprising a self assembled monolayer (SAM). Depending on the molecular structure of the SAM, the thickness may be between a few tens of angstroms up to around 10 nm or even more than 20 nm. Bound to the surface of the SAM is schematically shown a bio-receptor molecule 230.

In typical operation of the device, a test fluid is passed over the bio-receptors bound to the SAM. The test fluid typically comprises a liquid saline solution. The bio-receptors are designed to specifically bind to a target bio-molecule, or to a bio-molecular group, and the SAM is designed to bind the bioreceptors and to avoid non-specifically binding to other molecules. If and only if the test fluid contains molecules or molecular groups compatible with the bio-receptors (that is, if it contains the targeted molecules or biomarkers), the molecules will bind to the bio-receptors via a biological affinity reaction. This has the effect of changing the electrical properties of the transistor 201. In particular, the impedance path, inside the cavity - comprising the counter electrode, fluid (typically saline solution), bound molecule, bioreceptor and SAM - will be modified by the presence of the target bio-molecule bound to the bio-receptor, which can be sensed by the transistor 201. Thus by electrical probing of the transistor 201, the presence of the target bio-molecule in the fluid may be established. Furthermore by more sophisticated electrical analysis, it can be possible to determine not only the presence of, but also the concentration of the target bio-molecule.

Figure 3 shows an example of a SAM bonded onto a copper electrode according to an embodiment of the invention. Note that the electrode may have dimensions of the order of microns or even of the order of nanometres, but the invention is not so limited. Thus hereinafter and hereinbefore the term 'micro-electrode' is used as an alternative to the terms 'electrode' or 'nano-electrode' but is not be construed as conferring a specific dimensional limitation on the electrode.

The SAM 300 comprises individual molecules 302 containing alkane chains which are aligned or assembled in the same orientation. (In the figure, the orientation is shown as vertical.) As shown in figure 3, the molecule 302 comprises four distinct parts 311, 312, 313 and 314. Although alkane chains are preferred, in other embodiments, or carbon-containing chains can be used in place of the alkane chain.

The first part 311 is a bonding group, and as shown is a thiol group (-SH). The thiol group is in direct contact with the copper electrode 301, and provides a strong chemical bond to the copper electrode 301. In other embodiments of the invention, an alternative to the thiol group is provided, and in particular, a selenol group (-SeH) may be provided as the alternative.

The second part of the molecule 302 is a carbon-based chain R1 shown at 312. The carbon-based chain, R1 is between the bonding group 311 and and the optional part 313 which will be described below. Preferably, and as shown in figure 3, the carbon chain is saturated, and thus comprises an alkane. In less preferred embodiments, the alkane chain may be interrupted by one or more other carbon-based groups, or even by non-carbon hetero atoms. An example of an other carbon-based group is an alkyne or an alkene group, such that the carbon chain is unsaturated. An example of such a group including a hetero-atom is an alkoxy group.

A characterising feature of the carbon-based chain R1 is that it is hydrophobic. Furthermore, the SAM according to the invention is well packed. In other words, the molecules are closely spaced together. This is significant, since it is necessary in order for this part of the SAM to accomplish its purpose of isolating the copper surface from the solution to avoid copper oxidation. Generally it is possible to more closely pack together molecules which have saturated alkyl chains without any side chains, than chains including either or both one or more side chains or alkene or alkyne groups, or even branched chains or hetero-atoms.

The third part 313 of the molecule 302 comprises a polyethylene-oxide (PEO) monomer. This monomer has the chemical formula -(O-CH₂-CH₂)ₘ. It acts to reduce non-specific adsorption of bio-molecules onto the SAM, by means of decreasing the electrostatic/hydrophobic interactions between the surface and the analytes, as will be well known to the skilled person. PEO chains are hydrophilic. Furthermore, PEO groups are less stiff than alkane chain so the top layer of the SAM is more flexible to increase the bioreceptor coupling efficiency. If only alkyl chains without any PEO were to be used, unwanted molecules (which can be different from the target molecule is it desired to bind) could adhere to the SAM upper surface by electrostatic adsorption, hydrophobic interactions or other interactions. The presence of the PEO monomer 313, can thus improve the specificity of the biosensor. However, for some applications it is not required, and in particular, some bio-receptors are sufficiently specific to their target bio-molecule such as not to require the PEO monomer.

The optional part 313 of PEO monomer is between the carbon-based chain 312 and a chemical terminal group 314. In the embodiments which do not include monomer 313, the carbon-based chain 312 is directly connected to the chemical terminal group 314.

The role of the chemical terminal group 314 is to form a chemical bond with a bio-receptor. Importantly, in order to ensure binding of the bio-receptor molecules, the terminal group 314 should be a hydrophilic. In the embodiment shown in figure 3, the terminal group comprises a carboxylic acid group (COOH). However, in other embodiments the terminal group can be other hydrophilic groups such as, without limitation, the hydroxyl group (-OH), the aldehyde group (-CHO), biotin, the cyclic ether, or the amine (-NH2). Two characterising features of the terminal group are that: firstly, it must be possible to be activated in order to provide a binding site for a bio-receptor; and secondly the group must ensure that the SAM, or at least the end of the SAM distal from the copper electrode, is hydrophilic in order that binding can occur.

An example of a SAM which has been used according to embodiments of the invention is16-mercapto-hexadecanoic acid. This molecule, also known as "16-MHA", has chemical formula HS-(CH₂)₁₅-COOH where n=15. The bonding group comprises a thiol group (-SH), the carbon-based chain comprises pentadecyl -(CH₂)₁₅-, there is no PEO part, and the terminal group comprises carboxylic acid (-COOH) .

The SAM 300 is deposited and bonded to the copper electrode 301, by any suitable means, as will be known to the skilled person. Prior to deposition of the SAM, the copper is cleaned and the oxide is removed.

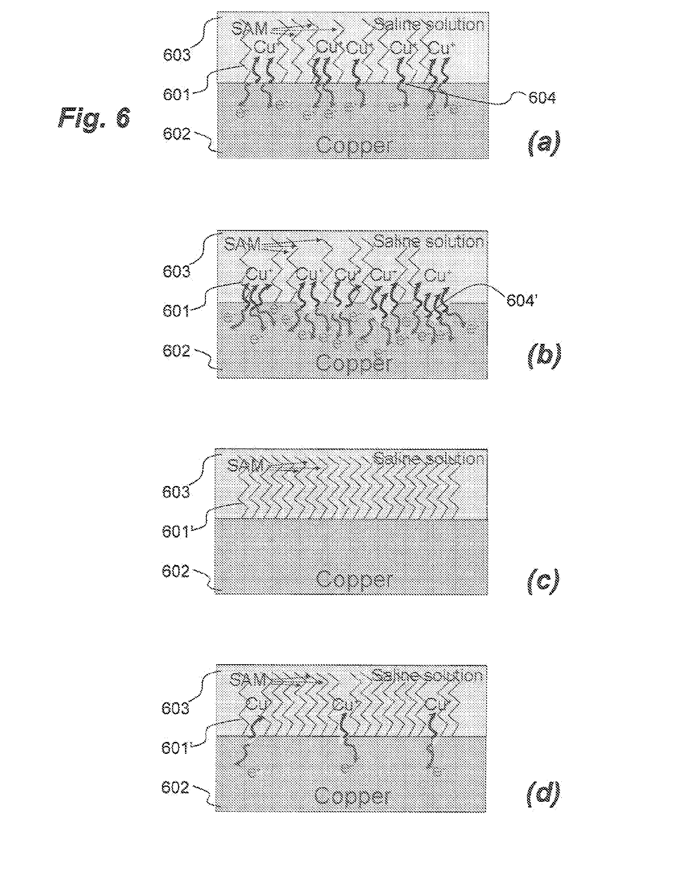

Figure 6 shows a schematic representation of a test measurement of a SAM's robustness to electrochemical stress. A saline solution 603 (typically Phosphate Buffer Saline solution, containing NaCl) is injected into the biosensor cavity, above the surface of the copper 602. To provide electrochemical stress, an Ag/AgCl counter electrode is then immersed into this electrolyte. Figure 6(a) shows a poorly deposited, loosely packed SAM 601 at the start of the test. During the test, copper in contact with the solution will be progressively oxidized and then corroded, especially at the interface 604 between two Copper grains. These cationic sites will expand during the measurement and resulting holes will further degrade the SAM, leading to an expanding corrosion. By the end of the test, as depicted at Figure 6(b), both the copper surface and the SAM are significantly degraded. In contrast, a properly deposited SAM, well attached and densely packed - as shown at Figure 6(c) at the start of the test - will not allow significant contact between the saline solution and the copper, and thus still efficiently protect copper from corrosion at the end of the measurement as shown at Figure 6(d).

According to embodiments of the invention, a bio-receptor molecule is bonded or anchored to the SAM. In order to allow this, a functional group such as COOH is mandatory for these embodiments.

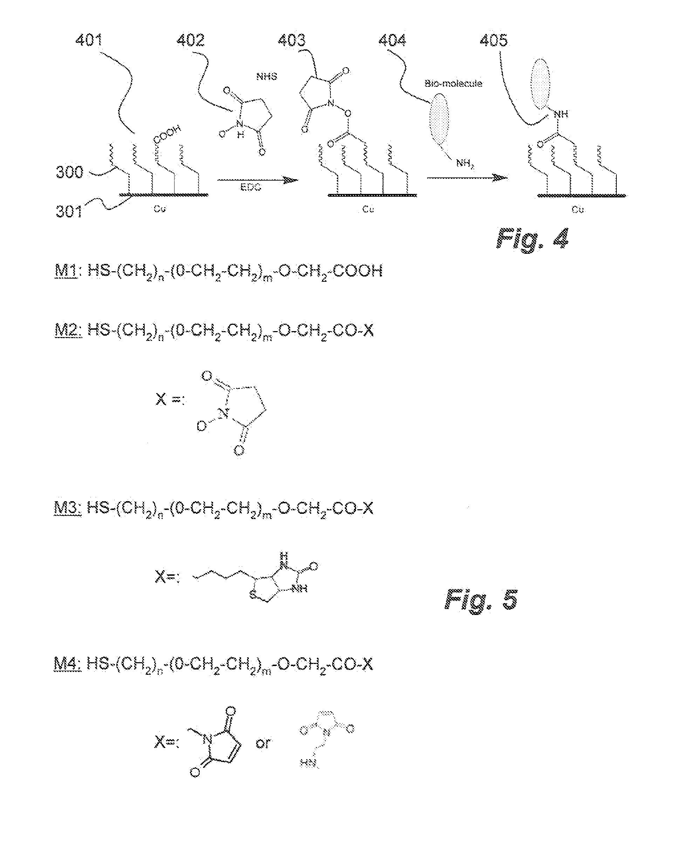

A first method of anchoring a bio-receptor according to the invention is illustrated in Figure 4, and comprises the following sequential steps:
- at 401, deposition of an appropriate SAM 300 such as HS-(CH₂)n-(O-CH₂-CH₂)m-O-CH₂-COOH including, for instance, the carboxylic acid group COOH as the head group, onto copper electrode 301;
- activation of the COOH active group by an intermediate cross-linker 402 which forms the intermediate Molecules M2 or M3 (403) attached to the copper, where X can be the N-hydroxide succinimide (NHS) or the biotin hydrazide as shown in Figure 5 or 2-(2-pyridinyldithio)-ethane amine (PDEA) or molecules with the general formula: maleimide-R-NH₂, or other suitable precursor for a bio-receptor. For this step, ethyl dimethylaminopropyl carbodiimide (EDC) activator compound is used to chemically activate the - COOH group (forming intermediate compound) and allow the cross-linker such as NHS to bind to the COO⁻; and
- coupling of the desired bio-receptor 404 in saline buffer to result in a coupled bio-receptor molecule 405.

In other embodiments, the SAM is assembled from molecules which are already activated, and thus directly ready to have a bio-receptor bonded, or anchored, to the terminal group. In this case the SAM is deposited from preactivated thiol or selenol molecules. Non-limiting examples of such preactivated molecules are N-hydroxide succinimide -ester and biotinylated thiol molecules.

As used in this application, the term "bio-sensor" and "bio-sensing" are not to be construed narrowly, but the skilled person will appreciate that they encompass biological interface chemistry, in general. Of significance is the fact that the relate to essentially *biological* interactions.

Furthermore, the skilled person will appreciate that the phrase "self assembled monolayer" (SAM) as used in this application has its usual meaning - that is the layer itself comprising a monolayer of molecules, which molecules have a head group with a special affinity for a substrate, and a tail including a functional group, as defined for instance in Wikipedia. The phrase is not to be read as to being constrained as to the details of deposition or the method of constructing the monolayer.

In summary, seen from one viewpoint, then, a device for bio-sensing applications is disclosed, comprising a substrate such as a semiconductor chip having Cu electrodes thereon, and a self assembled monolayer bonded to at least one of the Cu electrodes, wherein molecules of the self-assembled monolayer comprise a head group which bonds to Cu, a carbon-comprising chain comprising a chain of at least 11 C atoms, and a terminal group which is hydrophilic and for binding a bio-receptor. The terminal group is hydrophilic to allow binding to the bio-receptor, and inclusion of the carbon-comprising chain, limits or avoids corrosion of the copper.

Also disclosed is a method of providing such a device, activating the terminal group and coupling a bio-receptor to the activated terminal group. Disclosure further extends to use of such a device for bio-sensing applications. The term may be considered as synonymous with "self-assembling monolayer", although herein the more widely accepted phrase for SAM has been used.

From reading the present disclosure, other variations and modifications will be apparent to the skilled person. Such variations and modifications may involve equivalent and other features which are already known in the art of biosensors, and which may be used instead of, or in addition to, features already described herein.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination.

The applicant hereby gives notice that new claims may be formulated to such features and/or combinations of such features during the prosecution of the present application or of any further application derived therefrom.

For the sake of completeness it is also stated that the term "comprising" does not exclude other elements or steps, the term "a" or "an" does not exclude a plurality and reference signs in the claims shall not be construed as limiting the scope of the claims.

## Claims

1. A device for bio-sensing applications, comprising
a substrate having Cu electrodes thereon,
and a self assembled monolayer bonded to at least one of the Cu electrodes,
wherein molecules of the self-assembled monolayer comprise a head group which bonds to Cu,
a carbon-comprising chain comprising a chain of at least 11 C atoms, and
a terminal group which is hydrophilic and for binding a bio-receptor.

2. A device as claimed in claim 1, wherein molecules of the self-assembled monolayer further comprise at least one polyethylene oxide group between the alkyl chain and the terminal group.

3. A device as claimed in claim 1 or 2, wherein the molecules of the self-assembled monolayer are selected from the group consisting in HS-R1-Y and HS-R1-(-O-CH₂-CH₂-)ₘ-Y, HSe-R1-Y and HSe-R1-(-O-CH₂-CH₂-)ₘ-Y, where m is either a positive integer or 0.

4. A device as claimed in claim 3, where Y is selected from the group consisting in -(COOH), -(OH), -(CHO), -biotin, -cyclic ether, and -(NH₂),

5. A device as claimed in claim 3 or 4, wherein R1 is a carbon-comprising chain of n carbon atoms, interrupted by p hetero-atoms, where n and p are each positive integers.

6. A device as claimed in claim 3 or 4, wherein R1 is a carbon-comprising chain of n carbon atoms without interruption, where n is a positive integer.

7. A device as claimed in claim 5 or 6, where R1 comprises an alkyl, alkenyl, cyclic alkyl, aryl, alkyl bound to aryl, alkenyl bound to aryl or alkynyl bound to aryl.

8. A device as claimed in claim 5, wherein R1 is an alkyl group.

9. A device as claimed in any of claims 5 to 8 wherein n is an integer greater than 10.

10. A device as claimed in any of claims 5 to 8, wherein n is an integer between 13 and 19.

11. A sensor comprising a device as claimed in any preceding claim, wherein the sensor further comprises a bio-receptor bonded to the terminal group.

12. A sensor as claimed in claim 11, wherein the bio-receptor is bound to the terminal group by means of a cross-linker, which cross-linker is selected from the group consisting in 1-Ethyl-3-[3-dimethylaminopropyl]-carbodiimide hydrochloride, N-hydroxide succinimide, biotin hydrazide, 2-(2-pyridinyldithio)-ethane amine (PDEA) and Maleimide-R-NH₂ and their derivatives containing polyethylene oxide units.

13. A method of forming a biosensor comprising providing a device as claimed in any of claims 1 to 10, activating the terminal group, and coupling a bio-receptor to the activated terminal group by means of a cross-linker.

14. A method as claimed in claim 13, wherein the coupling is effected in a buffer solution.

15. A method as claimed in claim 13 or 14, wherein the cross-linker is selected from the group consisting in N-hydroxide succinimide, biotin hydrazide, 2-(2-pyridinyldithio)-ethane amine (PDEA) and Maleimide-R-NH₂ and their derivatives containing polyethylene oxide units.

16. Use of a sensor as claimed in claim 11 or claim 12, to detect a predetermined biologically active target molecule or a predetermined biologically active target functional group.

17. Use as claimed in claim 16, where the respective active target molecule or molecular group is in a saline solution.

## Amended claims

### Amended claims in accordance with Rule 137(2) EPC.

**1.** A device for bio-sensing applications, comprising
a substrate having Cu electrodes thereon,
and a self assembled monolayer bonded to at least one of the Cu electrodes,
wherein molecules of the self-assembled monolayer comprise a head group which is one of a thiol group and a selenol group and which bonds to Cu,
a carbon-comprising chain comprising a chain of at least 11 C atoms, and
a terminal group which is hydrophilic and for binding a bio-receptor.

**2.** A device as claimed in claim 1, wherein molecules of the self-assembled monolayer further comprise at least one polyethylene oxide group between the alkyl chain and the terminal group.

**3.** A device as claimed in claim 1 or 2, wherein the molecules of the assembled monolayer are selected from the group consisting in HS-R1-Y and HS-R1-(-O-CH₂-CH₂-)ₘ-Y, HSe-R1-Y and HSe-R1-(-O-CH₂-CH₂-)ₘ-Y, where m is either a positive integer.

**4.** A device as claimed in claim 3, where Y is selected from the group consisting in -(COOH), -(OH), -(CHO), -biotin, -cyclic ether, and -(NH₂),

**5.** A device as claimed in claim 3 or 4, wherein R1 is a carbon-comprising chain of n carbon atoms, interrupted by p hetero-atoms, where n and p are each positive integers.

**6.** A device as claimed in claim 3 or 4, wherein R1 is a carbon-comprising chain of n carbon atoms without interruption, where n is a positive integer.

**7.** A device as claimed in claims 5 or 6, where R1 comprises an alkyl, alkenyl, cyclic alkyl, aryl, alkyl bound to aryl, alkenyl bound to aryl or alkynyl bound to aryl.

**8.** A device as claimed in claim 5, wherein R1 is an alkyl group.

**9.** A device as claimed in any of claims 5 to 8 wherein n is an integer greater than 10.

**10.** A device as claimed in any of claims 5 to 8, wherein n is an integer between 13 and 19.

**11.** A sensor comprising a device as claimed in any preceding claim, wherein the sensor further comprises a bio-receptor bonded to the terminal group.

**12.** A sensor as claimed in claim 11, wherein the bio-receptor is bound to the terminal group by means of a cross-linker, which cross-linker is selected from the group consisting in 1-Ethyl-3-[3-dimethylaminopropyl]-carbodiimide hydrochloride, N-hydroxide succinimide, biotin hydraxide, 2-(2-pyridinyldithio)-ethane amine (PDEA) and Maleimide-R-NH₂ and their derivatives containing polyethylene oxide units.

**13.** A method of forming a biosensor comprising providing a device as claimed in any of claims 1 to 10, activating the terminal group, and coupons a bio-receptor to the activated terminal group by means of a cross-linker.

**14.** A method as claimed in claim 13, wherein the coupling is effected in a buffer solution.

**15.** A method as claimed in claim 13 or 14, wherein the cross-linker is selected from the group consisting in N-hydroxide succinimide, biotin hydrazide, 2-(2-pyridinyldithio)-ethane amine (PDEA) and Maleimide-R-NH₂ and their derivatives containing polyethylene oxide units.

**16.** Use of a sensor as claimed in claim 11 or claim 12, to detect a predetermined biologically active target molecule or a predetermined biologically active target functional group.

**17.** Use as claimed in claim 16, where the respective active target molecule or molecular group is in a saline solution.
